# EUROPEAN PATENT SPECIFICATION

(11) **EP 1 582 602 B1**
(45) Date of publication and mention of the grant of the patent: **16.02.2011**
(21) Application number: 05006619.0
(22) Date of filing: 24.03.2005
(51) Int. Cl.: C22C 9/00, C22F 1/08, H01B 1/02

(54) **Copper alloy and copper alloy manufacturing method**
Kupferlegierung und Verfahren zur Herstellung dieser Kupferlegierung
Alliage de cuivre et son procédé de fabrication

(30) Priority: 29.03.2004 JP 2004096654
(43) Date of publication of application: 05.10.2005
(73) Proprietor: NGK Insulators, Ltd., Nagoya-shi, Aichi-ken (JP); AKIHISA INOUE, Sendai-city, Miyagi-ken 980-0861 (JP)
(72) Inventor: Kimura, Hisamichi, Miyagi-ken, 989-2311 (JP); Muramatsu, Naokuni, Legal Affairs & Intellectual Property Department, Nagoya-shi Aichi-ken (JP); Suzuki, Ken, Legal Affairs & Intellectual Property Department, Nagoya-shi Aichi-ken (JP)
(74) Representative: TBK-Patent

(56) References cited:
- JP-A- 4 165 055
- JP-A- 6 346 206
- JP-A- 7 011 363
- JP-A- 7 054 079
- JP-A- 10 183 274
- JP-A- 57 009 850
- JP-A- 62 240 732
- US-A- 4 067 750
- US-A- 4 749 548
- US-A- 4 755 235
- US-A- 5 149 498
- US-A- 5 306 465

## Description

### BACKGROUND OF THE INVENTION

### 1. Field of the Invention

The present invention relates to a copper alloy having both tensile strength and electric conductivity, which is utilized mainly for connectors and sockets, and a method of manufacturing the same.

### 2. Description of the Related Art

A strip, foil, sheet, rod and the like made of a copper alloy which is press-molded/stamping has been used for general electronic parts and the like. In designing such a copper alloy, a copper alloy is desired to have both tensile strength and electric conductivity, which are contradicting properties. Furthermore, a copper alloy used for electronic parts loaded onto automobiles is desired to have a heat-resistance property in addition to the tensile strength and electric conductivity.

Cu-Zr based alloys are well known as heat resistance lead frame materials. Cu-Zr based alloys increase their heat-resistance property by solid solution of a small amount of Zr into Cu. In Cu-Zr based alloys, Zr supersaturated at a high temperature generates a stabilized compound together with Cu with an ageing treatment, thus precipitating. As a result, Cu-Zr based alloys increase their tensile strength (refer to Patent No. 2501275, for example).

However, when Zr is added unnecessarily, a problem arises in that the electric conductivity is decreased. Further, coarse crystals and precipitations which do not contribute to the tensile strength of Cu-Zr based alloys remain, and have a possibility to role as original points of cracks at the time when the Cu-Zr based alloys are press-molded/stamping to make electronic parts such as connectors.

Therefore, the addition of Zr which is considered to be proper has been set roughly from 0.005 to 0.25% in terms of weight ratio (refer to Japanese Patent Laid-Open No. H10 (1998) -183274, for example).

However, in such Cu-Zr based alloys, a combination of a tensile strength σ (ultimate tensile strength σ) and an electric conductivity 5 are σ: 300 MPa-δ: 90% IACS ("Precipitation of Alloy" by Nariyasu Kohda, Maruzen, pp 442, Fig. 13. 3, issued on July 25, 1972).

Herein, in designing the copper alloy, a dimensionless performance index value M = σ(MPa)*δ(% IACS) is defined to indicate a balance between the tensile strength σ and the electric conductivity δ, which contradict each other. The dimensionless performance index value M of conventional Cu-Zr based alloys remained approximately 270 (300*0.9). Since the conventional Cu-Zr based alloys have a small value of the dimensionless performance index value M, its applicable scope is limited. When a property including both tensile strength σ and electric conductivity δ is required, the conventional Cu-Zr based alloys can not be applicable.

Accordingly, as a copper alloy having a property in that both the tensile strength σ and the electric conductivity δ are high, the copper alloy satisfying an inequality expressed by M > 400 is eagerly desired.

On the other hand, in order to increase the value of the dimensionless performance index value M of a Cu-Zr based alloy, the addition of a third element has already been tried.

For example, in Japanese Patent Laid-Open No. H10 (1998)-183274, several copper alloys having the dimensionless performance index value M equal to about 600*0. 7 = 420 have been proposed by adding a small amount of many elements such as Cr and Zn to a Cu-Zr based alloy.

However, exemplification of an alloy achieving the dimensionless performance index value M more than 400 while having a higher tensile strength σ (high strength) does not exist. Therefore, there has been a problem in that such a Cu-Zr based alloy is used only for an extremely limited application.

Furthermore, when a Cu-Zr based alloy ends its nominal life as an electronic parts and when it is reused as re-melted raw materials, there is a problem in that maintenance of such multiple system alloy scraps is extremely troublesome.

### BRIEF SUMMARY OF THE INVENTION

In light of the foregoing problems, the inventors of this application examined properties of thin strip having a thickness of 30 to 50 µm, which are respectively made of Cu₉₅Zr₃M₂ (M: Ti, V, Nb, Cr, Mn, Fe, Co, Ni, or Al) and Cu₁₀₀-ₓZrₓ (x: 0 to 5) by use of a single roll type casting method which is one way to obtain an amorphous thin strip by casting.

As a result, the inventors of this application found that in Cu-5%Zr (by atomic percent), a multiphase structure composed of a Cu matrix and a Cu₉Zr₂ compound is obtained, and that a grain diameter of the Cu matrix is extremely small ("Copper and Copper Alloy", Vol. 42, No. 1, pages 193 through 197, issued in 2003).

However, the tensile strength σ (ultimate tensile strength σ) of the copper alloy was about 1080 MPa, and its electric conductivity δ was about 24% IACS (% of International Annealed Copper Standard). A dimensionless performance index value M of the copper alloy remained at about 259 (1080*0.24).

Thereafter, the inventors of this application found a copper alloy in which a composition by atomic percent is expressed by a compositional formula of Cu₁₀₀ - _{(a + b)}ZrₐB_{b} 1.0 ≤ a ≤8.0, 0 ≤ b ≤ 4.0, and a + b ≤ 8.0), and completed the present invention.

An object of the present invention is to provide a copper alloy which has a dimensionless performance index value M satisfying an inequality M > 400, and which can be applied to a wide range of electronic parts, in a simple alloy composition of a binary system containing Cu and Zr or of a ternary system containing Cu, Zr, and B. Further, Another object of the present invention is to provide a method of manufacturing the same.

In order to achieve the foregoing objects, a first aspect of the present invention is a copper alloy according to claim 1 in which a composition by atomic percent is expressed by a composition formula: Cu₁₀₀ - _{(a+b)}ZrₐB_{b}, with a dual phase structure, layered with a plurality of Cu matrices constituted by grain particles having an average diameter of 10 µm or less and with a eutectic phase constituted by the Cu matrices and any of a Cu-Zr compound and a Cu-Zr-B compound. As for the grain particles, at least a part of each of the grain particles contacts other adjacent grain particles, and in Cu₁₀₀ - _{(a + b)} ZrₐB_{b}, "a", "b" and " (a + b)" satisfy 1.0 ≤ a ≤ 8.0, 0 ≤ b ≤ 4.0, and a + b ≤ 8.0. is the copper alloy

A second aspect of the present invention is the copper alloy according to the first aspect, in which in the dual phase structure, a precipitation containing at least one of a Cu-Zr compound, a Cu-B compound and a Cu-Zr-B compound is dispersed in the grain particles.

A third aspect of the present invention is a method of manufacturing the copper alloy according to the first through second aspects of the present invention, the method comprising the steps of melting and casting the copper alloy by a non refractory melting method (which is a method of melting without refractory crucible), and performing a cold working with a reduction of 50% or more for the copper alloy.

A fourth aspect of the present invention is the method according to the third aspect of the present invention, the method further comprising the step of performing a thermal treatment to the copper alloy at a temperature in a range from 550 to 800°C and for 1 to 5 hours, immediately before the step of performing the cold working.

A fifth aspect of the present invention is the method according to the third or fourth aspect of the present invention, the method further comprising the step of performing an ageing treatment for the copper alloy at a temperature in a range from 300 and 500°C and for 1 to 10 hours, after the step of performing the cool working.

According to the foregoing aspects of the present invention, a dual phase structure constituted by a Cu matrix and either or both of a Cu-Zr-B compound and a Cu-Zr compound is obtained around the fine grain particles of the Cu matrix, and a sufficient tensile strength can be acquired.

When an average diameter of the grain particles of the Cu matrix is no more than 10 µm, the dual phase structure can be effectively obtained, and the number of grain particles contacting other grain particles and the total contact area of the grain particles increase. Therefore, the tensile strength and the electric conductivity of the copper alloy are increased.

In the dual phase structure, the precipitation containing at least one of a Cu-Zr compound, a Cu-B compound and a Cu-Zr-B compound is dispersed in the grain particles, whereby the tensile strength is further increased.

Specifically, according to the foregoing aspects of the present invention, the copper alloy can be provided, which can be applied to a wide range corresponding to the application of electronic parts, and which is excellent in the tensile strength, electric conductivity and heat-resistance property. Furthermore, the method of manufacturing the copper alloy can be provided.

The copper alloy according to the foregoing aspects of the present invention is constituted by a simple binary system alloy composition containing Cu and Zr or by a simple ternary system alloy composition containing Cu, Zr and B. Therefore, it is possible to efficiently conduct maintenance of such a copper alloy when it is reused as re-melted raw materials in the form of alloy scraps after electronic parts using the copper alloy end its nominal life.

### BRIEF DESCRIPTION OF THE DRAWINGS

Fig. 1 is a graph showing measured results of a tensile strength and an electric conductivity versus an addition ratio of Zr, in a Cu-Zr based alloy according to a first embodiment of the present invention;
Fig. 2 is a graph showing measured results of a tensile strength and an electric conductivity versus an addition ratio of Zr, in a Cu-Zr-B based alloy according to a second embodiment of the present invention;
Fig. 3A is schematic views of as-cast structure of the copper alloy according to the first and second embodiment;
Fig. 3B is schematic views of a structure the copper alloy which has been cold-rolled after casting;
Fig. 3C is schematic views of a structure of the copper alloy which has been subjected to an ageing treatment after the casting and cold rolling;
Fig. 4 is a schematic view of a typified structure of a conventional Cu-Zr based alloy;
Fig. 5 is a graph showing a dimensionless performance index value M of a Cu-Zr based alloy containing the copper alloy according to the first embodiment; and
Fig. 6 is a graph showing a dimensionless performance index value M of a Cu-Zr-B based alloy containing the copper alloy according to the second embodiment.

### DESCRIPTION OF THE PREFERRED EMBODIMENT

A copper alloy according to this embodiment, which has both of the tensile strength and electric conductivity, has a composition by atomic percent being expressed by a composition formula: Cu₁₀₀ᵢ - _{(a + b)} ZrₐB_{b}. The copper alloy according to this embodiment constitutes a dual phase structure having a layered structure formed by a plurality of Cu matrices constituted by grain particles and by an eutectic phase constituted by the Cu matrices and any of a Cu-Zr compound and a Cu-Zr-B compound.

At least part of each of the grain particles constituting the Cu matrix contacts other adjacent grain particles. In the composition formula: Cu₁₀₀ - _{(a + b)} ZrₐB_{b}, "a", "b" and "(a + b) satisfy 1.0 ≤ a ≤ 8.0, 0 ≤ b ≤ 4.0, and a + b ≤ 8.0.

An average diameter of the grain particles in the copper alloy according to this embodiment is about 10 µm or less.

In a dual phase structure of the copper alloy according to this embodiment, a precipitation containing at least one of a Cu-Zr compound, a Cu-B compound and a Cu-Zr-B compound is dispersed in the grain particles.

A method of manufacturing the copper alloy according to this embodiment comprises the steps of melting and casting copper alloy by a nonrefractory melting method, and performing cold working with a reduction of about 50% or more for the copper alloy after the step of melting and casting the copper alloy.

Preferably, the method of manufacturing copper alloy according to this embodiment further comprises the step of performing a thermal treatment for the copper alloy at a temperature between 550°C and 800°C and for 1 to 5 hours, immediately before the step of performing the cold working.

Preferably, the method of manufacturing copper alloy according to this embodiment further comprises the step of performing an ageing treatment for the copper alloy at a temperature between 300°C and 500°C and for 1 to 10 hours, after the step of performing the cold working.

The embodiments of the present invention will be described concretely hereinafter with reference to the accompanying drawings.

### (Constitution of copper alloy according to this embodiment and properties thereof)

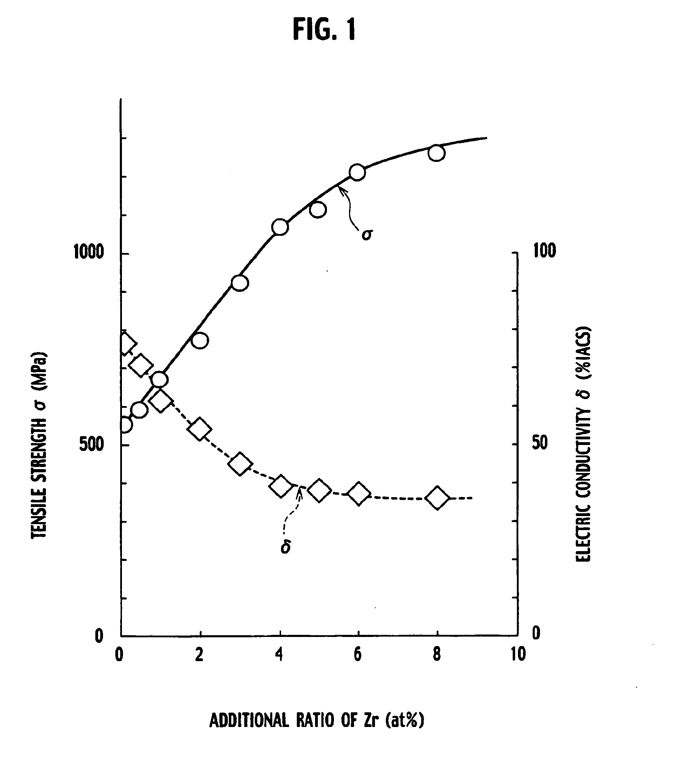

Fig. 1 is a graph showingmeasurement results of the tensile strength σ (ultimate tensile strength σ. hereinafter referred to as tensile strength σ) and electric conductivity δ versus an additional ratio of Zr, in a Cu-Zr based alloy according to a first embodiment of the present invention.

In this embodiment, a Cu-Zr based alloy was melted by levitation melting which is one of the nonrefractory melting methods. Subsequently, the melted Cu-Zr based alloy was cast by use of a Cu mold, and a plate-shaped bulk specimen having a thickness of about 10 mm was prepared.

Further, after the plate-shaped bulk specimen was cut to a proper dimension, the specimen was subjected to a cold rolling with a yield of 90% to 98%, thus making a sheet having a thickness of about 0.2 mm. The measured results of the tensile strength σ and electric conductivity δ for the sheet are shown in Fig. 1.

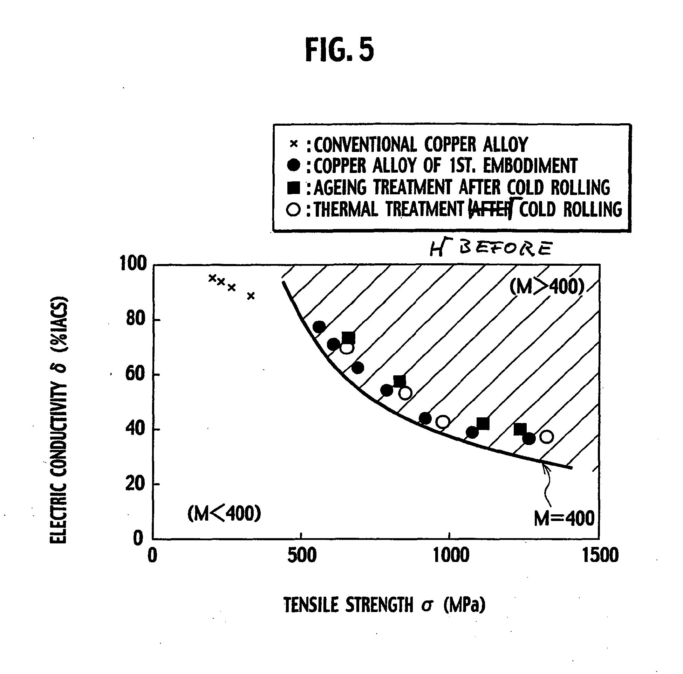

Values for a dimensionless performance index value M, which are determined based on the tensile strength σ and electric conductivity δ, are indicated by the symbols "•" in Fig. 5.

In Fig. 5, the symbols "■" indicate the measured results in the cases where an ageing treatment was performed for the specimen at 450°C for two hours after the cold working (cold rolling).

Furthermore, the symbols "o" indicate the measured results in the cases where a thermal treatment was performed for the specimen at 650°C for one hour before the cold working (cold rolling).

The curve in Fig. 5 indicates a combination of the tensile strength σ and electric conductivity δ in which the dimensionless performance index value M is 400. In addition, the symbols "x" indicate the measured results obtained for the conventional copper alloy, which are described in "Precipitation of Alloy" by Nariyasu Kohda, Maruzen, pp 442, Fig. 13. 3, issued on July 25, 1972.

As shown in Fig. 5, the dimensionless performance index value M of more than 400 which has not been heretofore achieved, can be obtained over a wide range (slant line portion in Fig. 5) in this embodiment.

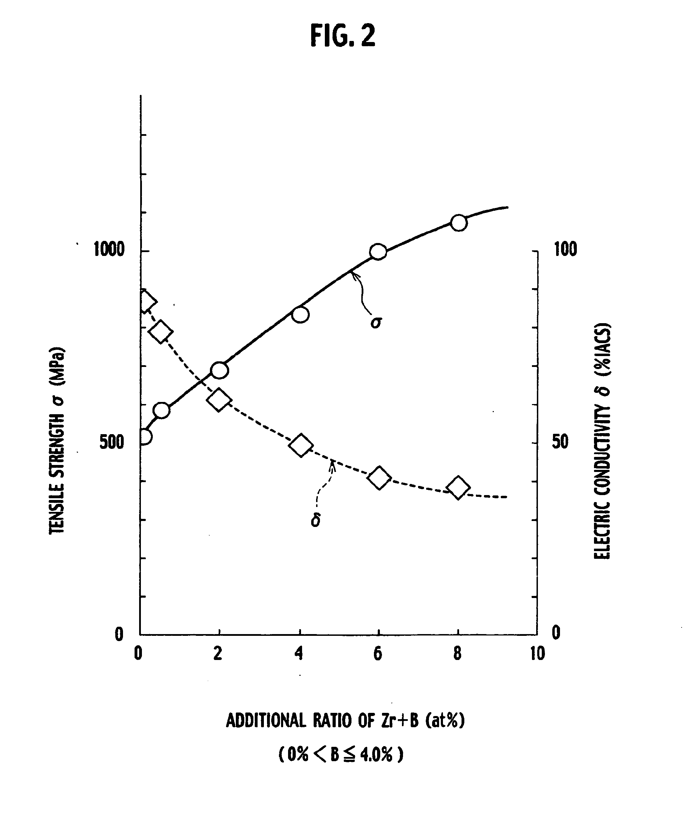

Fig. 2 is a graph showing measurement results of the tensile strength σ and electric conductivity δ versus an addition ratio of Zr, in a Cu-Zr-B based alloy according to a second embodiment of the present invention.

In this embodiment, the Cu-Zr-B based alloy was melted by a vacuum arc melting method which is one of the nonrefractory melting methods. Subsequently, the melted Cu-Zr-B based alloy was cast by use of a Cu mold, and a plate-shaped bulk specimen having a thickness of about 25 mm was prepared.

Furthermore, after the plate-shaped bulk specimen was cut to a proper dimension, the specimen was subjected to cold rolling with a reduction of 90% to 97%, thus making a sheet having a thickness of about 0.2 mm. The measured results of the tensile strength σ and electric conductivity δ for the sheet are shown in Fig. 2.

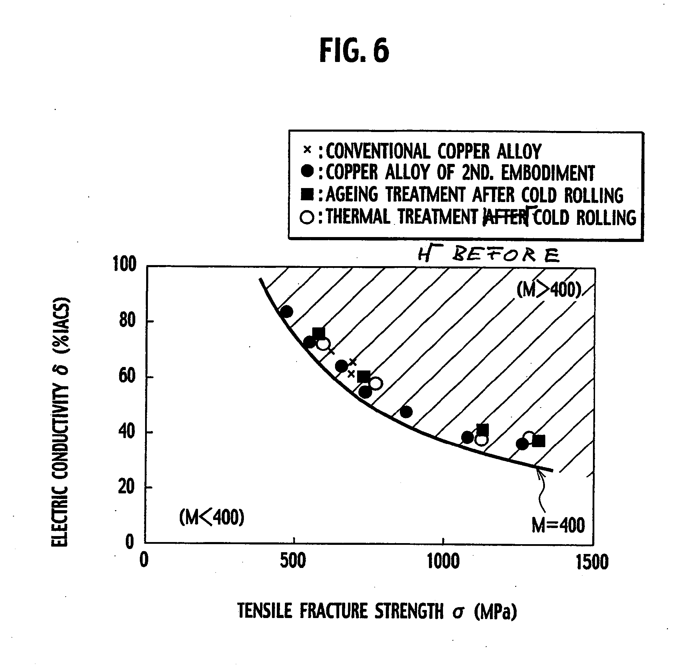

Values for a dimensionless performance index value M determined based on the tensile strength σ and electric conductivity δ are indicated by the symbols "●" in Fig. 6.

In Fig. 6, the symbols "■" indicate the measured results in the cases where an ageing treatment was performed for the specimen at 460°C for two hours after the cold working (cold rolling).

Furthermore, the symbols "'o" indicate the measured results in the cases where a thermal treatment was performed for the specimen at 650°C for one hour before the cold working (cold rolling).

The curve in Fig. 6 indicates a combination of the tensile strength σ and electric conductivity δ in which the dimensionless performance index value M is 400. In addition, the symbols "x" indicate the measured results obtained for the conventional copper alloy, which are described in Japanese Patent Laid-Open No. H10(1998)-183274.

As shown in Fig. 6, the dimensionless performance index value M of more than 400 can be obtained over a wide range (slant line portion in Fig. 6) in this embodiment.

Herein, the nonrefractory melting method used for manufacturing the copper alloy according to this embodiment is not limited to the levitation melting and the vacuum arc melting, but electron beam melting can be used.

The shape of the bulk specimen prepared by casting is not limited to the plate shape, as the specimen may be a round bar, a block or a pipe by contriving an appropriate mold.

The cold working does not need to be limited to the rolling, since it is possible to select all working methods capable of obtaining stretching of the dual phase structure, the methods include extrusion, drawing, forging, pressing and the like.

The atmosphere and the cooling means are not particularly limited in the thermal treatment before the cold working and the ageing treatment after the cold working. Note that the thermal treatment and the ageing treatment should be preferably performed in the presence of an inert gas such as argon and nitrogen or in vacuum in order to reduce forming of oxidation scales.

Air-cooling and water-cooling should be preferably adopted as the cooling method when a reduction in working hours is considered.

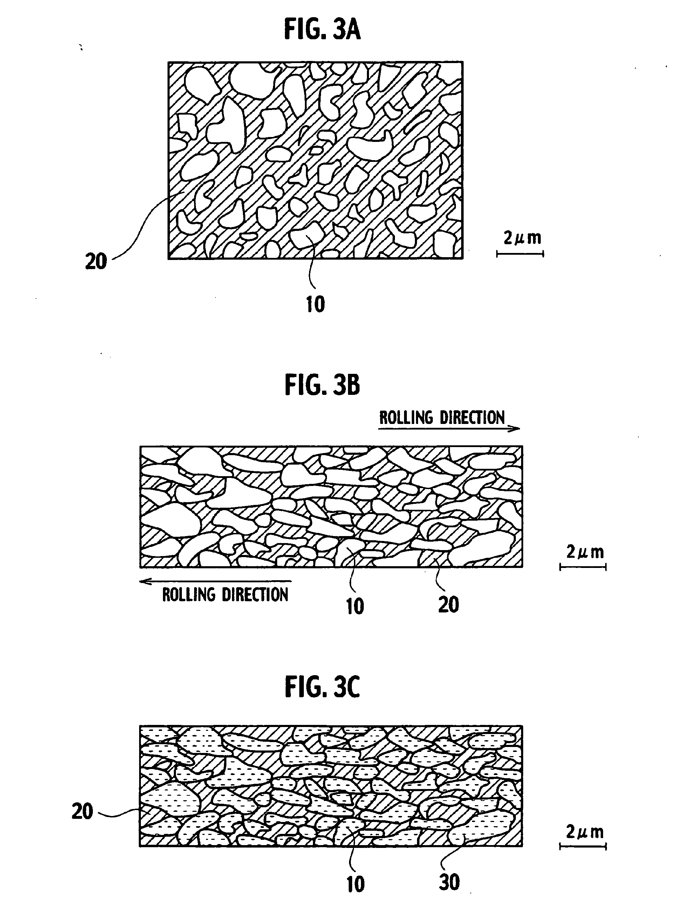

Figs. 3A, 3B and 3C are schematic views showing a structure of the copper alloy according to the first and second embodiments. The copper alloy shown in Fig. 3A has an isotropic Cu matrix 10 in which the average diameter of the grain particles is about 10 µm or less.

The copper alloy shown in Fig. 3A constitutes a dual phase structure having a layered structure including the Cu matrix 10, and an eutectic phase 20 constituted by a Cu-Zr compound essentially containing Cu₉Zr₂ and a Cu-Zr-B compound.

Fig. 3B shows a structure at the time when the copper alloy shown in Fig. 3Ahas been subjected to cold rolling with a reduction of 90% after the casting. Grain particles constituting the Cu matrix 10 are stretched in the direction the copper alloy is processed.

In the eutectic phase 20, a shearing-sliding deformation aptly occurs, and the dual phase structure becomes layered. At the same time, a part of the grain particle comes to contact other grain particles.

Further, Fig. 3C shows a structure at the time when the copper alloy shown in Fig. 3B has been subjected to the ageing treatment. As shown in Fig. 3C, precipitation 30 constituted by any of Cu-Zr compound, Cu-B compound and Cu-Zr-B compound is precipitated in the grain particles.

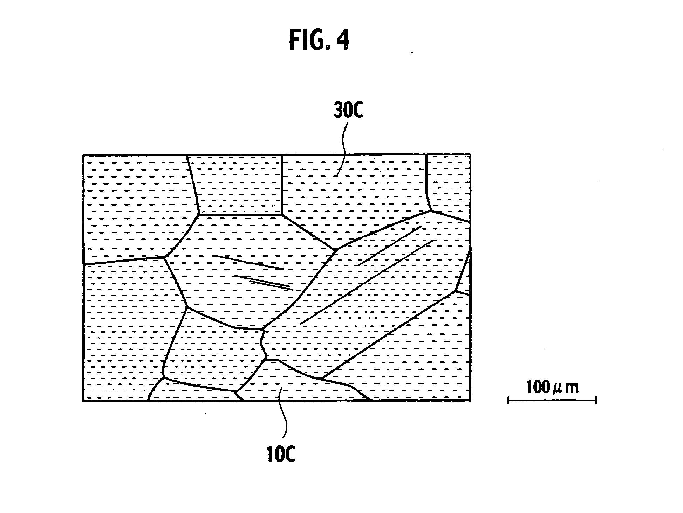

Fig. 4 is a schematic view of a typified structure of a Cu-Zr based alloy having Cu matrix 10C and precipitation 30C. The Cu-Zr based alloy is obtained by a conventional manufacturing method (melting and casting, hot rolling, solution-heat treatment, cold rolling, and ageing treatment) have been performed sequentially, as an example of a comparison with the copper alloy of the first and second embodiments shown in Figs. 3A to 3C.

The reason why a value "a" of Zr in the copper alloy expressed by the composition formula: Cu₁₀₀ - _{(a + b)} ZrₐB_{b} by atomic percent, in which 1.0 ≤ a ≤ 8.0, 0 ≤ b ≤ 4.0 and a + b ≤ 8.0 is defined as follows. Specifically, when the value "a" of Zr is too small than a range expressed in the above inequalities in this embodiment, a desired structure is not formed, and the strength of the copper alloy cannot be obtained.

On the other hand, when the value "a" of Zr is too large, a decrease in the electric conductivity δ is brought about. As a result, not only the dimensionless performance index value M is less than 400, but also cold workability is disturbed.

Boron (B) in the copper alloy, without decreasing the electric conductivity δ, plays a role in constituting the structure by compensating Zr, and contributes to an improvement in the strength. Furthermore, the boron (B) suppresses coarsening of the grain particles, and improves the cold workability.

When the boron (B) is added to an amount beyond the foregoing range, the cold workability is disturbed. Incidentally, Japanese Patent Official Gazette No. 2501275 reports that the addition of the boron (B) disturbs the cold workability.

The grain particles constituting the Cu matrix should preferably have an average diameter of about 10 µm or less. When the average diameter of the grain particles is no more than 10 µm, it is possible to obtain the foregoing dual phase structure effectively.

According to the manufacturing method of this embodiment, the diameter of the grain particles is inevitably no more than 10 µm.

It is supposed that the Cu matrix is tentatively prepared so as to have the diameter of the grain particles larger than 10 µm, by means other than that defined in the manufacturing method according to this embodiment. In this case, it is anticipated that a volume ratio of the eutectic phase promoting the stretching of the dual phase structure, which occupies in the total volume of the dual phase structure decreases, and the shearing-sliding deformation of the eutectic phase is not performed smoothly.

Further, since the size of the grain particle is large, the number of grain particles contacting other grain particles and the total area of the surfaces of the grain particles contacting other grain particles decrease even if the dual phase structure is stretched by the cold working.

Accordingly, the increase in electric conductivity δ is not expected, and it is impossible to obtain the dimensionless performance index value M of more than 400.

The reason why the cold working is performed with the reduction of about 50% or more is as follows. It is difficult to obtain a layered structure when the reduction is below 50%.

If the layered structure is not obtained, the total area of the surfaces of the grain particles contacting other grain particles becomes small, and the electric conductivity δ decreases. Accordingly, the dimensionless performance index value M of more than 400 cannot be obtained.

In this embodiment, the thermal treatment prior to the cold working in the manufacturing method of the copper alloy is performed for one to five hours at a temperature in a range from 550 to 800°C. If the temperature of the thermal treatment is below 500°C, diffusion of atoms is insufficient, and re-solving of the compound does not occur.

On the other hand, if the temperature of the thermal treatment is higher than 800°C, coarsening of the crystal becomes undesirably significant.

If the treatment duration is shorter than one hour, it is insufficient to allow the compound to re-melt. If the treatment duration is longer than five hours, such treatment duration is not only inefficient, but also undesirable because such long treatment duration may lead to coarsening of the crystal grains depending on the treatment temperature.

In this embodiment, the ageing treatment after the cold working in the manufacturing method of the copper alloy is performed for one to ten hours at a temperature in a range from 300 to 500°C. If the temperature of the ageing treatment is below 300°C, precipitation of the Cu-Zr compound, the C-B compound or the Cu-Zr-B compound does not advance, and an effect of increasing the tensile strength of the copper alloy cannot be expected.

On the other hand, if the temperature of the ageing treatment is higher than 500°C, a part of the compound begins to melt into the matrix, and the tensile strength σ decreases again.

An ageing duration is determined depending on the temperature, and if the ageing duration is shorter than one hour, an improvement in the tensile strength σ is not expected. If the ageing duration is longer than ten hours, precipitation becomes coarse. The tensile strength σ is not only improved, but also ineffectiveness is brought about because of the long ageing duration.

In the foregoing first and second embodiments of the present invention, the manufacturing method capable of suppressing mixing of elements, which disturb the improvements in tensile strength σ and electric conductivity δ by producing impurity substances such as oxide and sulfide containing oxygen (O), sulfur (S) and silicon (Si), is used. In other words, in the manufacturing method, a particular casting method such as a single rolling casting method showing a limitation in the size of manufacturing products is not used.

To be concrete, in order to suppress the mixing amount of the elements disturbing the improvements in the tensile strength σ and electric conductivity δ to be 1,000 ppm or less, the copper alloy was melted by use of the nonrefractory melting method. Subsequently, the melted copper alloy was cast at a solidification rate at which the bulk was obtained, by use of the Cu cast mold.

The dual phase structure in which around the fine grain particles (Cu matrix) having an average diameter of no more than 10 µm, the precipitation containing at least one of the Cu-Zr compound, the Cu-B compound and the Cu-Zr-B compound is dispersed in the grain particles can be obtained, and the sufficient tensile strength σ can be secured.

Furthermore, in the first and second embodiments, the cold working with the reduction of about 50% or more is performed for the bulk which has been cast, whereby the eutectic phase is allowed to cause the shearing-sliding deformation preferentially.

When the eutectic phase causes the shearing-sliding deformation, the eutectic phase stretches in a direction where the dual phase structure is worked, and the Cu matrix also deforms and stretches. Therefore, a part of the grain particle comes to contact other grain particles, and such a contact of the grain particle with other grain particle promotes smooth flowing of free electrons, thus improving the electric conductivity δ.

Herein, by adding the boron (B), it is possible to easily obtain the eutectic phase and to improve the workability of the copper alloy. When the workability of the copper alloy is improved, the total contact area of the grain particles increases, and the electric conductivity δ can be further increased.

### (Other Embodiments)

Next, other embodiments of the copper alloy according to the present invention will be described. Table below shows a composition of the specimen according to the embodiments. In the embodiments, as the specimen, the specimens of the embodiments 1 through 17, the specimens of the comparison examples 1 through 6, and the specimens of the conventional example 7 through 11 (described in the literature) were used.

The melting methods,etc.,such assolidification material (solidification mode), refractory, cast dimension, treatment before working, yield of cold working and ageing treatment, are also shown.

The copper alloys according to the embodiments 1 through

| | COMPOSITION (at%) | MELTlNG METHOD | SOLIDIFICATION FORM | REFRACTORY | CAST DIMENSION | TREATMENT BEFORE WORKING | REDUCTION OF COLD WORKING | AGEING TREATMENT |
|---|---|---|---|---|---|---|---|---|
| | | | | | THICKNESS(mm) | TEMP.XDURATI0N | % | TEMPXDURATION |
| EMBODIMENT 1* | Cu-0.1Zr | NONREFRACTORY | BULK | - | to | - | 98 | - |
| EMBODIMENT 2 | Cu-1.0Zr | NONREFRACTORY | BULK | - | 10 | - | 98 | - |
| EMBODIMENT 3 | Cu-3.0Zr | NONREFRACTORY | BULK | - | 25 | - | 95 | - |
| EMBODIMENT 4 | Cu-5.0Zr | NONREFRACTORY | BULK | - | 25 | - | 90 | - |
| EMBODIMENT 5 | Cu-8.0Zr | NONREFRACTORY | BULK | - | 10 | - | 85 | - |
| EMBODIMEMT 6 | Cu-8.0Zr | NONREFRACTORY | BULK | - | 10 | 650°CX1h | 95 | - |
| EMBODIMENT 7 | Cu-5.0Zr | NONREFRACTORY | BULK | - | 10 | - | 97 | 450°CX2h |
| EMBODIMENT 8 | Cu-1.0Zr | NONREFRACTORY | BULK | - | 10 | 650°CX1h | 98 | 450°CX2h |
| EMBODIMENT 9 | Cu-8.0Zr | NONREFRACTORY | BULK | - | 10 | - | 55 | - |
| EMBODIMENT 10* | Cu-0.1Zr-0.058 | NONREFRACTORY | BULK | - | 25 | - | 97 | - |
| EMBODIMENT 11 | Cu-1.0Zr-0.3B | NONREFRACTORY | BULK | - | 25 | - | 90 | - |
| EMBODIMENT 12 | Cu-2.0Zr-1.0B | NONREFRACTORY | BULK | - | 10 | - | 90 | - |
| EMBODIMENT 13 | Cu-4.0Zr-3.98 | NONREFRACTORY | BULK | - | 10 | - | 88 | - |
| EMBODIMENT 14 | Cu-3.0Zr-2.0B | NONRETRACTORY | BULK | - | 25 | 650°CXth | 97 | - |
| EMBODIMENT 15 | Cu-3.0Zr-2.0B | NONREFRACTORY | BULK | - | 25 | - | 90 | 460°CX2h |
| EMBODIMENT 16 | Cu-3.0Zr-2.0B | NONREFRACTORY | BULK | - | 25 | 650°CX1h | 92 | 460°CX2h |
| EMBODIMENT 17 | Cu-3.0Zr-2.0B | NONREFRACTORY | BULK | - | 25 | - | 53 | - |
| COMPARISON EXAMPLE 1 | Cu-0.03Zr | NONREFRACTORY | BULK | - | 10 | - | 96 | - |
| COMPARISON EXAMPLE 2 | Cu-8.3Zr | NONREFRACTORY | BULK | - | 10 | - | ROLLING IMPOSSIBLE | - |
| COMPARISON EXAMPLE 3 | Cu-3.0Zr | CRUCIBLE | BULK | ALUMINA | 10 | - | 95 | - |
| COMPARISON EXAMPLE 4 | Cu-3.0Zr | NONREFRACTORY | BULK | - | 10 | 820°CX1h | 95 | - |
| COMPARISON EXAMPLE 5 | Cu-3.0Zr | NONREFRACTORY | BULK | - | 10 | - | 95 | 600°CX2h |
| COMPARISON EXAMPLE 6 | Cu-3.0Zr | NONREFRACTORY | BULK | - | 10 | - | 44 | - |
| CONVENTIONAL EXAMPLE 7 | Cu-0.023Zr | CRUCIBLE | BULK | ALUMINA | CAST BLOCK | 900°CX0,5h | 95 | 550°CX1h |
| CONVENTIONAL EXAMPLE 8 | Cu-0.17Zr-0.3Cr | NONREFRACTORY | BULK | CARBON | 30mm | 900°CX0.5h | 90 | - |
| CONVENTIONAL EXAMPLE 9 | Cu-5.0Zr | SINGLE ROLLER | FOIL | QUARTZ | 30µm | - | - | - |
| CONVENTIONAL EXAMPLE 10 | Cu-3.0Zr-2.0Cr | NONREFRACTORY | BULK | QUARTZ | 30µm | - | - | - |
| CONVENTIONAL EXAMPLE 11 | Cu-3,0Zr-2,0T1 | NONREFRACTORY | BULK | QUARTZ | 30µm | - | - | - |

| | | | | | | | | |
|---|---|---|---|---|---|---|---|---|
| * Embodiments 1,10 do not fall under the scope of the present invention | | | | | | | | |

The copper alloys according to the embodiments 1 through 17 are obtained by melting and casting by use of the nonrefractory melting method. To be concrete, in the embodiments 1, 2, 5, 6, 7, 8, 9, 12 and 13, the levitation melting is used. In the embodiments 3, 4, 10, 11, 14, 15, 16 and 17, the vacuum arc melting is used.

For the copper alloys according to the embodiments 1, 2, 3, 4, 5, 9, 10, 11, 12, 13 and 17, the thermal treatment and the ageing treatment before and after the cold working are not performed. On the other hand, for the copper alloys according to the embodiments 8 and 16, both of the thermal and ageing treatments are performed.

For the copper alloys according to the embodiments 6 and 14, only the thermal treatment before the cold working is performed. For the copper alloys according to the embodiments 7 and 15, only the ageing treatment after the cold working is performed.

The cold working of the copper alloys was performed by rolling. Further, the thermal treatment and ageing treatment were performed in the presence of nitrogen in an electric furnace. The air cooling was adopted as the cooling method.

Table below shows presence or absence of the two-phase structure, presence or absence of the layered structure, the grain diameter of the matrix, the tensile strength σ, the electric conductivity δ and the dimensionless performance index value M for each specimen.

Herein, the "dual phase structure" means a dual phase structure in which a part of a grain particle constituting the Cu matrix contacts other grain particles. Furthermore, the "layered structure" means a layered structure formed of the Cu matrix and the eutectic phase composed of a Cu matrix and at least one of the Cu-Zr compound and the Cu-Zr-B compound.

| | DUAL PHASE STRUCTURE | LAYERED STRUCTURE | GRAIN DIAMETER OF MATRIX | σ | δ | M |
|---|---|---|---|---|---|---|
| | | | µm | MPa | %IACS | σ X δ |
| EMBODIMENT 1* | YES | YES | 4 | 540 | 76 | 410 |
| EMBODIMENT 2 | YES | YES | 3 | 670 | 64 | 428 |
| EMBODIMENT 3 | YES | YES | 2 | 900 | 45 | 405 |
| EMBODIMENT 4 | YES | YES | 3 | 1,100 | 38 | 418 |
| EMBODIMENT 5 | YES | YES | 2 | 1,250 | 37 | 475 |
| EMBODIMENT 6 | YES | YES | 2 | 1,230 | 40 | 492 |
| EMBODIMENT 7 | YES | YES | 3 | 1,120 | 41 | 459 |
| EMBODIMENT 8 | YES | YES | 2 | 690 | 67 | 462 |
| EMBODIMENT 9 | YES | YES | 3 | 1,100 | 37 | 407 |
| EMBODIMENT 10 | YES | YES | 5 | 490 | 86 | 421 |
| EMBODIMENT 11 | YES | YES | 3 | 650 | 67 | 435 |
| EMBODIMENT 12 | YES | YES | 2 | 790 | 61 | 481 |
| EMBODIMENT 13 | YES | YES | 2 | 1,090 | 39 | 425 |
| EMBODIMENT 14 | YES | YES | 2 | 1,120 | 38 | 425 |
| EMBODIMENT 15 | YES | YES | 2 | 1,120 | 40 | 448 |
| EMBODIMENT 16 | YES | YES | 2 | 1,120 | 41 | 458 |
| EMBODIMENT 11 | YES | YES | 4 | 1,100 | 38 | 418 |
| OOMPARISON EXAMPLE 1 | NO | NO | 40 | 350 | 95 | 332 |
| COMPARISON EXAMPLE 2 | YES | NO | 4 | NO DATA | NO DATA | NO DATA |
| COMPARISON EXAMPLE 3 | NO | NO | 20 | 400 | 85 | 340 |
| COMPARISON EXAMPLE 4 | YES | YES | 30 | 800 | 40 | 320 |
| COMPARISON EXAMPLE 5 | YES | YES | 15 | 820 | 41 | 385 |
| COMPARISON EXAMPLE 6 | YES | NO | 12 | 880 | 30 | 264 |
| CONVENTIONAL EXAMPLE 7 | NO | NO | 300 | 310 | 90 | 279 |
| CONVENTIONAL EXAMPLE 8 | NO | NO | 380 | 450 | 77 | 346 |
| CONVENTIONAL EXAMPLE 9 | YES | NO | <1 | 1,080 | 24 | 259 |
| CONVENTIONAL EXAMPlE 10 | NO | NO | 25 | 670 | 5 | 33 |
| CONVENTIONAL EXAMPLE 11 | NO | NO | 30 | 780 | 5 | 39 |

| | | | | | | |
|---|---|---|---|---|---|---|
| * Embondiments 1,10 do not fall under the scope of the present invention | | | | | | |

In order to judge the constitution of the structure, an observation by use of an electron microscope was mainly performed for the copper alloys according to the embodiments. Note that an observation by use of an optical microscope was performed complementarily. Furthermore, for the copper alloys according to the comparison examples, an observation by use of the optical microscope was performed.

The tensile strength σ of the specimen was obtained by a tensile test, and the electric conductivity δ was obtained in such a manner that after measuring an electric resistance of the specimen at room temperature, a ratio (% IACS) of the measured electric resistance to that of pure copper was calculated.

As shown in the table, the copper alloys according to the embodiments 1 through 17 have the dual phase structure and the layered structure, and the tensile strength σ thereof ranges from 490 to 1250 MPa. In addition, the electric conductivity δ of the copper alloys ranges from 37 to 86% IACS, and the dimensionless performance index value M ranges from 405 to 492.

Specifically, the copper alloys achieve the dimensionless performance index value M of more than 400, and have both the tensile strength σ and electric conductivity δ.

On the other hand, the copper alloys according to the comparison examples 1 through 6 and the conventional examples 7 through 11 have the dimensionless performance index value M equal to 385 at a maximum, and cannot achieve the dimensionless performance index value M of more than 400. Specifically, it is understood that the copper alloys according to the embodiments 1 through 17 are excellent in the tensile strength σ and the electric conductivity δ.

Among the copper alloys according to the comparison examples, the copper alloy of the comparison example 4 showing a high dimensionless performance index value M, which is 320, and the copper alloy of the comparison example 5, which is 385, were made in the same manner as that for the copper alloy according to the embodiments.

The difference between the embodiments and the comparison examples 4, 5 is the temperature conditions of the thermal and ageing treatments before and after the cold working. Specifically, the copper alloy of the comparison example 4 was subjected, for one hour at 820°C, to the thermal treatment before the cold working. Further, the copper alloy of the comparison example 5 was subjected, for two hours at 600°C, to the ageing treatment after the cold working.

As described above, for the copper alloys of the comparison examples 4 and 5, the temperature of the thermal and ageing treatments before and after the cold working are higher than the temperature for the copper alloys of the embodiments. Owing to the high temperatures, the average diameter of the grain particles constituting the Cu matrix is 30 µm in the thermal treatment before the cold working.

Further, in the ageing treatment after the cold working, a re-precipitation of the compound containing Zr and B, which are solid-solved in the Cu matrix, is inhibited. Then, the average diameter of the grain particles becomes coarser beyond 10 µm.

Due to coarsening of the grain particles, the copper alloys of the comparison examples 4 and 5 have the tensile strength σ of 800 to 820 MPa and the electric conductivity 5 of 40 to 40% IACS. Therefore, the values for the dimensionless performance index value M become 320 to 385, not as high as M > 400.

On the other hand, in the embodiments 1 to 17, an average diameter of the grain particles constituting the Cu matrix is 2 to 5 µm, and it is understood that it is important that the average diameter of the grain particles is no more than 10 µm.

The difference of the dimensionless performance index value M depending on the presence or absence of the ageing treatment can be judged by comparing the copper alloys of the embodiments 4 and 7, or by comparing the copper alloys of the embodiments 14 and 16.

To be concrete, the dimensionless performance index value M of the copper alloys of the embodiments 7 and 16, which have been subjected to the ageing treatment, are larger than the copper alloys of the embodiments 4 and 14, which have not been subjected to the ageing treatment.

The reason why the dimensionless performance index values are different is that since solid-solution of the compound containing Zr and B in the Cu matrix occurs and the compound precipitates in the Cu matrix again, the tensile strength σ and the electric conductivity δ are further increased.

Specifically, it is understood that in the dual phase structure, the value of the dimensionless performance index value M increases by dispersing the precipitation containing at least one of the Cu-Zr compound, the Cu-B compound and the Cu-Zr-B compound in the grain particles.

Since the copper alloys according to the embodiments 1 through 17 do not cause re-solving until the ageing temperature exceeds 500°C, it is understood that these copper alloys also have excellent heat-resistance property.

Coincidentally, for the copper alloy of the comparison example 2, rolling was stopped because of the occurrence of significant cracks during rolling. Accordingly, it was impossible to measure the tensile strength σ, etc.

An object of the present invention is to provide copper alloy which has a dimensionless performance index value M satisfying an inequality: M > 400 in either a binary system alloy composition containing Cu and Zr, or a ternary system alloy composition containing Cu, Zr, and B. In the copper alloy of the present invention, a composition by atomic percent is expressed by a composition formula: Cu_{100 - (a+b)} ZrₐB_{b}, with a dual phase structure having a layered structure including a plurality of Cu matrices constituted by grain particles having an average diameter of 10 µm or less and a eutectic phase constituted by said Cu matrices and any of a Cu-Zr compound and a Cu-Zr-B compound. Part of each of the grain particles contacts other adjacent grain particles, and the "a", the "b", and the " (a + b) " satisfy 1.0 ≤ a ≤ 8.0, 0 ≤ b ≤ 4.0, and a + b ≤ 8.0.

## Claims

1. A copper alloy in which a composition by atomic percent is expressed by a composition formula: Cu_{100- (a+b)} ZrₐB_{b}, with
a dual phase structure having a layered structure formed by a plurality of Cu matrices constituted by grain particles having an average diameter of 10 µm or less and a eutectic phase constituted by said Cu matrices and any of a Cu-Zr compound and a Cu-Zr-B compound, the copper alloy **characterized in that**
at least part of each of said grain particles contacts other adjacent grain particles;
the "a", the "b" and the " (a + b)" satisfy 1.0 ≤ a ≤ 8.0, 0 ≤ b ≤ 4.0, and a + b ≤ 8.0; and
the dimensionless performance index value M > 400, wherein M is defined as M = σ(MPa)*S(% IACS), with σ being the ultimate tensile strength and S being the electric conductivity.

2. The copper alloy according to claim 1, wherein in said dual phase structure, a precipitation containing at least one of a Cu-Zr compound, a Cu-B compound and a Cu-Zr-B compound is dispersed in said grain particles.

3. A method of manufacturing the copper alloy according to claim 1 or 2, comprising the steps of:
melting and casting said copper alloy by a nonrefractory melting method; and
performing a cold working with a reduction of 50% or more for said copper alloy.

4. The method of manufacturing a copper alloy according to claim 3, the method further comprising the step of:
performing a thermal treatment for 1 to 5 hours at a temperature in a range from 550 to 800°C immediately before the step of performing said cold working.

5. The method of manufacturing the copper alloy according to claim 3 or 4, the method further comprising the step of:
performing an ageing treatment for 1 to 10 hours at a temperature in a range from 300 to 500°C after the step of performing said cold working.

## Patentansprüche

1. Kupferlegierung, in welcher eine Zusammensetzung nach Atomprozent durch die Zusammensetzungsformel ausgedrückt ist: CU_{100- (a+b)} ZrₐB_{b}, mit
einer Dualphasenstruktur mit einer geschichteten Struktur, die durch eine Mehrzahl von Kupfermatrizes, die durch Kornteilchen mit einem mittleren Durchmesser von 10 µm oder weniger konstituiert sind, und einer eutektischen Phase, die durch die Kupfermatrizes und einer aus einer Cu-Zr Verbindung und einer Cu-Zr-B Verbindung konstituiert ist, gebildet ist, wobei die Kupferlegierung **dadurch gekennzeichnet ist, dass**
zumindest ein Teil eines jeden der Kornteilchen andere benachbarte Kornteilchen kontaktiert;
das "a", das "b" und das "(a + b)" 1.0 ≤ a ≤ 8.0, 0 ≤ b ≤ 4.0, und a + b ≤ 8.0 erfüllen; und
der dimensionslose Leistungsindexwert M > 400, wobei M als M = σ (MPa) · δ (%IACS) definiert ist, mit σ als der Bruchfestigkeit und δ als der elektrischen Leitfähigkeit.

2. Kupferlegierung nach Anspruch 1, wobei in der Dualphasenstruktur eine Präzipitation, die zumindest eine aus einer Cu-Zr Verbindung, einer Cu-B Verbindung und einer Cu-Zr-B Verbindung enthält, in den Kornteilchen dispergiert ist.

3. Verfahren zur Herstellung der Kupferlegierung nach Anspruch 1 oder 2, das die Schritte umfasst:
Schmelzen und Gießen der Kupferlegierung durch ein nicht-refraktäres Schmelzverfahren; und
Ausführen einer Kaltbearbeitung mit einer Reduktion von 50 % oder mehr für die Kupferlegierung.

4. Verfahren zur Herstellung einer Kupferlegierung nach Anspruch 3, wobei das Verfahren ferner den Schritt umfasst:
Durchführen einer thermischen Behandlung für 1 bis 5 Stunden bei einer Temperatur in einem Bereich von 550 bis 800°C unmittelbar vor dem Schritt des Durchführens der Kaltbearbeitung.

5. Verfahren zur Herstellung der Kupferlegierung nach Anspruch 3 oder 4, wobei das Verfahren ferner den Schritt umfasst:
Durchführen einer Alterungsbehandlung für 1 bis 10 Stunden bei einer Temperatur in einem Bereich von 300 bis 500°C nach dem Schritt des Durchführens der Kaltbearbeitung.

## Revendications

1. Alliage de cuivre dans lequel une composition en pourcentage atomique est exprimée par la formule de composition : CU_{100- (a+b)} ZrₐB_{b}, avec
une structure double phase ayant une structure en couches formée par une pluralité de matrices de Cu constituées par des particules granulaires ayant un diamètre moyen de 10 µm ou moins et une phase eutectique constituée par lesdites matrices de Cu et l'un d'un composant Cu-Zr et d'un composant Cu-Zr-B, l'alliage de cuivre **caractérisé en ce que**
au moins une partie de chacune desdites particules granulaire se met en contact avec d'autres particules granulaires adjacentes ;
le "a", le "b" et le "(a + b)" satisfont 1,0 ≤ a ≤ 8,0, 0 ≤ b ≤ 4,0, et a + b ≤ 8,0 ; et
la valeur de l'indice de performance sans dimension M > 400, où M est défini comme M = δ (MPa)* δ (%IACS), δ étant la résistance finale à la traction et S étant la conductivité électrique.

2. Alliage de cuivre selon la revendication 1, dans lequel dans ladite structure double phase, une précipitation contenant au moins l'un d'un composé Cu-Zr, d'un composé Cu-B et d'un composé Cu-Zr-B est dispersée dans lesdites particules granulaire.

3. Procédé de fabrication de l'alliage de cuivre selon la revendication 1 ou 2, comprenant les étapes qui consistent :
à faire fondre et couler ledit alliage de cuivre par un procédé de fusion non réfractaire ; et
à réaliser un écrouissage avec une réduction de 50% ou plus pour ledit alliage de cuivre.

4. Procédé de fabrication d'un alliage de cuivre selon la revendication 3, le procédé comprenant en outre les étapes qui consistent :
à réaliser un traitement thermique pendant 1 à 5 heures à une température dans une plage de 550 à 800°C immédiatement avant l'étape qui consiste à réaliser ledit écrouissage.

5. Procédé de fabrication de l'alliage de cuivre selon la revendication 3 ou 4, le procédé comprenant en outre les étapes qui consistent :
à réaliser un traitement de vieillissement pendant 1 à 10 heures à une température se trouvant dans une plage de 300 à 500°C après l'étape qui consiste à réaliser ledit écrouissage.
